# EUROPEAN PATENT APPLICATION

(11) **EP 4 644 402 A1**
(43) Date of publication of application: **05.11.2025**
(21) Application number: 23912752.5
(22) Date of filing: 20.12.2023
(51) Int. Cl.: C07H 3/06, C07H 1/06

(54) **KESTOSE IN CRYSTALLINE FORM**

(30) Priority: 30.12.2022 KR 20220190878
(71) Applicant: Samyang Corporation, Jongno-gu Seoul 03129 (KR)
(72) Inventor: RYU, Kyunghun, Seongnam-si, Gyeonggi-do 13340 (KR); KIM, Goeun, Seongnam-si Gyeonggi-do 13434 (KR); PARK, Jiwon, Seongnam-si, Gyeonggi-do 13544 (KR); KIM, Dohyun, Seongnam-si, Gyeonggi-do 13504 (KR); HAN, Jungsook, Anyang-si, Gyeonggi-do 14062 (KR)
(74) Representative: Schön, Christoph
(86) International application number: PCT/KR2023/021094
(87) International publication number: WO 2024/144077

(57) **Abstract**

The present application relates to kestose in a crystalline form and a manufacturing method therefor.

## Description

### [TECHNICAL FIELD]

The present application relates to kestose in crystalline form.

### [BAKCGROUND ART]

Kestose is a type of fructooligosaccharide, and its efficacy has been confirmed in enhancing the production of immunoglobulin A (IgA) antibodies, inhibiting the production of immunoglobulin E (IgE) antibodies, promoting the proliferation of Bifidobacteria in the intestine, and improving atopic dermatitis in infants. Accordingly, it is industrially useful to efficiently produce kestose in order to utilize its utility as an allergy suppressing composition, allergy suppressing food and allergy suppressing agent using kestose.

However, powdery kestose is amorphous and highly hygroscopic, making it prone to stickiness, with low flowability and high sensitivity to ambient humidity. As a result, it tends to harden like candy during storage, making it very inconvenient to handle during product use. Therefore, there is a need for a technique for producing crystalline kestose particles that exhibit reduced hygroscopicity and improved flowability, allowing for improved handling similar to sugar.

### [DISCLOSURE]

### [TECHNICAL PROBLEM]

An embodiment of the present application is to provide crystalline kestose having a specific particle size distribution with reduced hygroscopicity and improved dissolution rate.

### [TECHNICAL SOLUTION]

An embodiment of the present application relates to crystalline kestose having a particle size distribution in which an average particle size represented by D(4,3) is 200 µm or more, and particles having a size of 70 µm or less account for 35% or less.

Another embodiment of the present application relates to a method for producing crystalline kestose, comprising a step of generating crystal nuclei at a temperature where the supersaturation degree of a kestose solution having a nystose content of less than 10 wt% based on a solid content of 100 wt% and a pH of 5 or higher, is more than 1 and less than 1.4; and a step of growing crystals.

Hereinafter, the present application will be described in further detail.

The crystalline kestose according to an embodiment of the present application has a low content of fine particles and a uniform particle size distribution. A lower content of fine particles and a more uniform size distribution of the crystals obtained in the kestose crystallization process can reduce inter-crystalline agglomeration, thereby decreasing hygroscopicity and enhancing the dissolution rates. In contrast, if the content of fine particles is high and the size uniformity is poor, inter-crystalline agglomeration is more likely to occur, resulting in greater hygroscopicity, slower dissolution, which makes adverse effects on product quality.

The crystalline kestose according to an embodiment of the present application has reduced hygroscopicity compared to the fine powder, and thus has characteristics of being stable during storage and easy to distribute and handle as it does not easily cake during storage, and has characteristics of being efficient in product use and being applicable to a wide range of fields as the dissolution rate is improved.

Specifically, the crystalline kestose according to an embodiment of the present application may have a particle size distribution in which the average particle size calculated from the particle volume and represented by D(4,3) is 200 µm or more, 250 µm or more, 280 µm or more, 300 µm or more, 340 µm or more, or 350 µm or more.

Specifically, the crystalline kestose according to an embodiment of the present application may have a particle size distribution in which the particles having a size of 70 µm or less account for 35% or less, 30% or less, 25% or less, 20% or less, 15% or less, 11% or less, 10% or less, 9% or less, 8% or less, 7% or less, 6% or less, or 5% or less.

Specifically, the crystalline kestose according to an embodiment of the present application may have a particle size distribution in which the particles having a size of 50 µm or less account for 20% or less, 15% or less, 10% or less, 7% or less, 5% or less, 4% or less, or 3% or less.

Specifically, the crystalline kestose according to an embodiment of the present application may have a particle size distribution in which the particles having a size of 10 µm or less account for 5.5% or less, 5% or less, 4% or less, 3% or less, 2% or less, 1.5% or less, 1.3% or less, 1.1% or less, 1% or less, 0.9% or less, 0.8% or less, 0.7% or less, 0.6% or less, 0.5% or less, or 0.4% or less.

Specifically, the crystalline kestose according to an embodiment of the present application may have a particle size distribution in which the particles having a size of 20 µm or less account for 7.5% or less, 7% or less, 6% or less, 5% or less, 4% or less, 3% or less, 2.5% or less, 2% or less, 1.5% or less, or 1% or less.

Specifically, the crystalline kestose according to an embodiment of the present application may have a particle size distribution in which the particles having a size of 50 µm or less account for 20% or less, 15% or less, 10% or less, 9% or less, 8% or less, 7% or less, 6% or less, 5% or less, 4% or less, or 3% or less.

Specifically, the crystalline kestose according to an embodiment of the present application may have a particle size distribution in which the particles having a size of 60 µm or less account for 25% or less, 20% or less, 15% or less, 10% or less, 9% or less, 8% or less, 7% or less, 6% or less, 5% or less, or 4% or less.

Specifically, the crystalline kestose according to an embodiment of the present application may have a particle size distribution in which the particles having a size of 80 µm or less account for 42% or less, 40% or less, 35% or less, 30% or less, 25% or less, 20% or less, 15% or less, 14% or less, 13% or less, 12% or less, 11% or less, 10% or less, 9.5% or less, 9% or less, 8% or less, 7% or less, or 6% or less.

Specifically, the crystalline kestose according to an embodiment of the present application may have a particle size distribution in which the particles having a size of 100 µm or less account for 45% or less, 40% or less, 35% or less, 30% or less, 25% or less, 20% or less, 15% or less, 13% or less, 12.5% or less, 12% or less, 11% or less, 10% or less, 9% or less, or 8% or less.

Specifically, the crystalline kestose according to an embodiment of the present application may have a particle size distribution where the particles having a size of 140 µm or less account for 55% or less, 50% or less, 45% or less, 40% or less, 35% or less, 30% or less, 29% or less, 28% or less, 27% or less, 26% or less, 25% or less, 24% or less, 23% or less, 22% or less, 21% or less, 20% or less, 19% or less, 18% or less, 17% or less, or 16.5% or less.

Specifically, the crystalline kestose according to an embodiment of the present application may have a particle size distribution where the particles having a size of 180 µm or less account for 60% or less, 55% or less, 50% or less, 45% or less, 42% or less, 40% or less, 39% or less, 38% or less, 37% or less, 36% or less, 35% or less, 30% or less, 29% or less, 28% or less, 27% or less, 26.5% or less, 26% or less, 25.5% or less, or 25% or less.

Specifically, the crystalline kestose according to an embodiment of the present application may have a particle size distribution where the particles having a size of 200 µm or less account for 65% or less, 60% or less, 55% or less, 54% or less, 53% or less, 52% or less, 51% or less, 50% or less, 49% or less, 48% or less, 47% or less, 46% or less, 45% or less, 44% or less, 43% or less, 42% or less, 41% or less, 40% or less, 39% or less, 38% or less, 37% or less, 36% or less, 35% or less, 34% or less, 33% or less, 32% or less, 31.5% or less, 31% or less, or 30.5% or less.

Specifically, the crystalline kestose according to an embodiment of the present application may have a particle size distribution where the particles having a size of 240 µm or less account for 78% or less, 75% or less, 70% or less, 69% or less, 68% or less, 67% or less, 66% or less, 65% or less, 60% or less, 55% or less, 50% or less, 49% or less, 45% or less, 40% or less, 39% or less, 38% or less, 37% or less, or 36% or less.

The crystalline kestose according to an embodiment of the present application may have at least one characteristic selected from the group consisting of the following (1) to (4):
(1) a melting temperature (Tm) of 206±5°C or 206±3°C,
(2) a melting enthalpy (△H) of 120±5 J/g or 120±3 J/g,
(3) a purity of 70 wt% or more, 80 wt% or more, 85 wt% or more, 90 wt% or more, 95 wt% or more, 96 wt% or more, 97 wt% or more, 98 wt% or more, or 99 wt% or more, and
(4) a nystose content of less than 1.4 wt%, 1 wt% or less, 0.9 wt% or less, 0.8 wt% or less, 0.7 wt% or less, 0.6 wt% or less, 0.5 wt% or less, 0.4 wt% or less, 0.3 wt% or less, 0.25 wt% or less, or 0.2 wt% or less.

Another embodiment of the present application relates to a method for producing crystalline kestose. The larger the average size of the kestose crystals, the better the physical properties and the greater the convenience of use. In order to produce such large-sized crystals, both the seed crystal process and the main crystallization process which are separated by a transfer process, must be performed. However, the method for producing kestose crystals according to an embodiment of the present application can produce large-sized kestose crystals at a high yield even in a single-step process.

A method for producing crystalline kestose according to an embodiment of the present application may include a step of producing a raw solution for kestose crystal; a step of controlling the supersaturation degree of the raw solution for kestose crystal to generate crystal nuclei; and a step of growing crystals. The raw solution for kestose crystal may be a product of kestose conversion reaction obtained by performing a kestose conversion. Specifically, the step of producing the raw solution for kestose crystal may include a step of performing a kestose conversion reaction from sucrose; a step of performing high-purity separation of kestose from a product of the kestose conversion reaction; and a step of optionally performing a filtration process and/or an ion-purification process.

Specific examples of the method of producing crystalline kestose may include a first ion-purification, SMB chromatography separation, a second ion-purification, concentration and crystallization processes, and optionally, the product of kestose conversion reaction may be subjected to an activated carbon treatment process, an ion-purification process, or both an activated carbon treatment process and an ion purification process.

The product of kestose conversion reaction may be produced using β-fructofuranosidase derived from Non-GMO *Aspergillus niger.* Specifically, sucrose is dissolved in water, heated to 40 to 65°C, and the pH is titrated to 6.5 to 7.0 using a 1.0 to 4.0 N sodium hydroxide solution, followed by addition of β-fructofuranosidase for reaction. Afterwards, when the kestose is generated to be 45 wt% or more of kestose content, the pH is titrated to 7.6 or higher, for example, pH 8.0, and then heated to 70 to 90°C to inactivate the enzyme. Afterwards, the mixture is filtered to remove colored substances by treating with 0.5 to 1.0% of activated carbon based on the solid content. After the filtration process, ion purification is performed. While the general purification process passes through K (cation) column - A (anion) column - MB column (Mixed Bed: K:A = 1:2), in the case of kestose, the A tower is installed at the end to increase the pH of the reaction solution, such as K-A towers or MB-A towers, MB-K-A towers, or K-MB-A towers. This is to prevent fructooligosaccharids such as kestose and nystose from being decomposed during the manufacturing process. Afterwards, the Brix is adjusted to 50 to 60 wt% through concentration, and then kestose is separated at a high purity using simulated moving bed (SMB) chromatograph as a chromatography high-purity separation process. Na⁺ type and Ca²⁺ type resin can be used at this time. The separated raw solution is obtained with a kestose content of 80 to 98 wt%. The high-purity separated kestose goes through an ion-purification process again. The high-purity kestose-containing solution that has passed through the SMB passes through an ion purification tower and has a pH of 5.0 to 8.0. Afterwards, it is concentrated to 70 wt% or more through a concentration process, and then a crystallization process can be performed.

The kestose solution for crystallization may have a solid content of 60 to 90 wt%, 60 to 85 wt%, 65 to 90 wt%, 65 to 85 wt%, 70 to 90 wt%, 70 to 85 wt%, 75 to 90 wt%, 75 to 85 wt%, 78 to 90 wt%, 78 to 85 wt%, 80 to 90 wt%, or 80 to 85 wt%.

The kestose solution for crystallization may be a high-purity kestose solution containing kestose in an amount of 85 wt% or more, for example, 90 wt% or more, based on 100 wt% of solid content.

The kestose solution for crystallization may have a nystose content of 10 wt% or less, less than 10 wt%, 9 wt% or less, 8 wt% or less, 7 wt% or less, 6 wt% or less, 5 wt% or less, 4 wt% or less, 3 wt% or less, 2 wt% or less, or 1 wt% or less, based on 100 wt% of solid content.

The kestose solution for crystallization may have a pH of 5 to 8, pH 5 to 7.5, pH 5.5 to 8, pH 5.5 to 7.5, pH 6 to 8, pH 6 to 7.5, pH 6.5 to 8, pH 6.5 to 7.5, pH 7 to 8, or pH 7.5 to 8. The lower the pH of the composition for kestose crystallization, the more likely it is that fructooligosaccharides will decompose during the process, lowering the purity, which may adversely affect the crystallization yield and crystal particles. On the other hand, if the pH is too high, browning, in which the color of the solution turns yellow, may become more severe. Therefore, it is preferable for crystallization to be performed at an appropriate pH condition.

A method for producing crystalline kestose according to an embodiment of the present application can perform the crystallization by controlling the temperature and/or concentration of a kestose concentrate solution. Specifically, a supersaturation state required for crystallization can be maintained by lowering the temperature of the kestose solution or changing the concentration of the kestose solution. In a specific embodiment of the present application, the progress of crystallization can be monitored by collecting samples at regular intervals in the crystallization step and observing them with the naked eye or a microscope, or analyzing the saccharide concentration and crystal particle shape of a supernatant obtained from centrifugation of the sample, and the temperature or the concentration of kestose can be controlled according to the result. In addition, the step of growing crystals may further include a step of dissolving fine crystals produced in the step of growing crystals one or more times.

The crystallization process can be performed in various ways, and the process can include a cooling method in which the temperature is cooled to form crystals in a supersaturated state, and/or an evaporative concentration method in which water is evaporated to increase the concentration and form crystals.

An example of the "cooling method" may be to induce crystal growth by controlling the cooling rate and temperature. For example, the cooling method may be to induce crystal growth by controlling the cooling rate and temperature without performing concentration under the reduced pressure. In order to grow the crystals well, it is important to control the cooling rate so that the supersaturation degree of the raw solution for crystallization is constantly maintained during the temperature cooling process. Therefore, the present prevention may include one or two sections in which the temperature is kept constant during cooling, and include cooling the temperature at a constant rate.

For example, the cooling method can induce a supersaturated state by cooling the kestose solution to a temperature of 75 to 30°C, 70 to 30°C, 65 to 30°C, 60 to 30°C, 55 to 30°C, 50 to 30°C, 45 to 30°C, 40 to 30°C, or 35 to 30°C, thereby forming crystals.

The cooling rate may be -0.1 to -5°C/hour, -0.1 to -3°C/hour, -0.1 to -2°C/hour, - 0.1 to -1.5°C/hour, -0.5 to -5°C/hour, -0.5 to -3°C/hour, -0.5 to -2°C/hour, -0.5 to - 1.5°C/hour, -1 to -5°C/hour, -1 to -3°C/hour, -1 to -2°C/hour, or -1 to -1.5°C/hour. If the cooling rate is low, the time of co-crystal formation is long, which may result in low productivity, and if the cooling rate is high, crystals of small particle size are formed, which may make it difficult to recover the crystals.

The method for producing the kestose crystal may include a step of generating crystal nuclei at a temperature where the degree of supersaturation of a high-purity kestose solution - containing 85 wt% or more of kestose, having a solid content of 60 to 90 wt% or preferably 78 to 85 wt%, and a pH in the range of 6 to 8 or preferably pH 6.5 to 7.5 - is more than 1 and 1.4 or less, or preferably 1.01 to 1.3; and a step of growing crystals by cooling the temperature of the solution.

Specifically, the method for producing the kestose crystal may include a step of slowly stirring a kestose-containing solution containing 85 wt% or more of kestose and having a solid content of 78 to 83 wt%, at a temperature of 50 to 70° C to generate crystal nuclei; and a step of cooling the temperature of the solution to 25 to 35 °C to grow crystals. The method for producing the kestose crystal may additionally include a step of adding a seed crystal.

The size of the seed crystal may be 50 to 500 µm, 50 to 450 µm, 50 to 400 µm, 50 to 350 µm, 50 to 300 µm, 100 to 500 µm, 100 to 450 µm, 100 to 400 µm, 100 to 350 µm, 100 to 300 µm, 150 to 500 µm, 150 to 450 µm, 150 to 400 µm, 150 to 350 µm, 150 to 300 µm, 200 to 500 µm, 200 to 450 µm, 200 to 400 µm, 200 to 350 µm, 200 to 300 µm, 250 to 500 µm, 250 to 450 µm, 250 to 400 µm, 250 to 350 µm, 250 to 300 µm, 300 to 500 µm, 300 to 450 µm, 300 to 400 µm, or 300 to 350 µm.

The amount of the seed crystal added may be 0.01 to 5 wt%, 0.01 to 3 wt%, 0.01 to 2 wt%, 0.01 to 1.5 wt%, 0.01 to 1 wt%, 0.1 to 5 wt%, 0.1 to 3 wt%, 0.1 to 2 wt%, 0.1 to 1.5 wt%, 0.1 to 1 wt%, 0.5 to 5 wt%, 0.5 to 3 wt%, 0.5 to 2 wt%, 0.5 to 1.5 wt%, 0.5 to 1 wt%, 1 to 5 wt%, 1 to 3 wt%, 1 to 2 wt%, or 1 to 1.5 wt%, based on the weight of the solid content of the crystallization solution.

An example of the "evaporative concentration method" may include a step of generating crystal nuclei, under vacuum state, at a temperature where the degree of supersaturation of a high-purity kestose solution - containing 85 wt% or more of kestose, having a solid content of 60 to 90 wt% or preferably 65 to 85 wt%, and a pH in the range of 6 to 8 or preferably pH 6.5 to 7.5 - is more than 1 and 1.4 or less, or preferably 1.01 to 1.3; and a step of growing crystals under vacuum and constant temperature conditions while maintaining the degree of supersaturation at 1.1 . A step of additionally adding the raw solution for crystallization may be included to stably maintain the supersaturation state. The step of additionally adding the raw solution for crystallization may be performed under constant temperature conditions, and that is, may not be accompanied by a decrease in temperature. If the temperature of the raw solution for kestose crystallization is lowered, the viscosity of the raw solution for crystallization increases, which reduces the fluidity of the raw solution and thus reduces collisions between molecules, which may affect crystal growth. In addition, as the viscosity increases, the stickiness becomes severe, which may make it difficult to recover the crystals. Additionally, if the viscosity of the crystal mother liquor is high, it becomes difficult to separate the crystals and the mother liquor, and more processes for washing are required, which ultimately has a negative effect on the recovery rate. If even a small amount of the mother liquor is attached to the crystals, the purity of the crystal particles may decrease, or the hygroscopicity may increase during storage, causing them to harden or caking. Therefore, in an example of the present application, the concentrating may not be accompanied by an increase in the supersaturation of the crystal mother liquor.

Specifically, the method for producing the kestose crystal includes a step of introducing, at a temperature of 60 to 70°C, a kestose-containing solution - containing 85 wt% or more of kestose and having a solid content of 60 to 90 wt% - into a reactor such that it occupies 20 to 50% of the reactor volume, and slowly stirring to generate crystal nuclei, and a step of growing crystals while maintaining a vacuum state and constantly maintaining the concentration at 60 to 90 wt%, 60 to 85 wt%, 60 to 83 wt%, 65 to 90 wt%, 65 to 85 wt%, 65 to 83 wt%, 70 to 90 wt%, 70 to 85 wt%, 70 to 83 wt%, 75 to 90 wt%, 75 to 85 wt%, 75 to 83 wt%, 80 to 90 wt%, 80 to 85 wt%, or 80 to 83 wt%. The maintaining the concentration constantly may be achieved by additionally adding the kestose-containing solution. Specifically, it may be performed by repeating the additional addition of the solution one to four times in the crystal growth process.

The method for producing the kestose crystals may additionally include a step of adding a seed crystal. The seed crystal is as described above.

In one specific embodiment of the present application, the method for producing the crystalline kestose includes a step of performing a second ion purification on a kestose fraction obtained in a high-purity separation process, a step of concentrating the ion-purified kestose fraction, a step of crystallizing kestose from the concentrate to obtain a kestose crystal, and optionally may additionally include the steps of recovering the kestose crystals, a washing process, and a drying process.

The method for producing kestose crystals according to the present application may further include a step of recovering the kestose crystals obtained in the crystallization step by various solid-liquid separation methods, for example, a step of centrifugation, a step of washing with deionized water, and a step of drying. The drying step may be performed in a fluidized bed dryer or a vacuum dryer, but is not limited thereto.

The crystallization yield of the method for producing crystalline kestose according to the present application may be 40% or more, 45% or more, 46% or more, 47% or more, 48% or more, 49% or more, 50% or more, 51% or more, 52% or more, 53% or more, 54% or more, 55% or more, or 56% or more.

The crystalline kestose according to an embodiment of the present application may have reduced hygroscopicity. For example, when the crystalline kestose is stored at a temperature of 25° C and a relative humidity of 75% for 6 hours, the hygroscopicity may be 2% or less, 1.95% or less, 1.9% or less, 1.85% or less, 1.8% or less, 1.75% or less, 1.7% or less, 1.65% or less, 1.6% or less, 1.55% or less, or 1.5% or less.

The crystalline kestose to an embodiment of the present application may have improved dissolution rate. For example, the time required to dissolve the crystalline kestose in water at a concentration of 10% (w/w) may be 250 seconds or less, 240 seconds or less, 230 seconds or less, 220 seconds or less, 210 seconds or less, 200 seconds or less, 190 seconds or less, 180 seconds or less, 170 seconds or less, 160 seconds or less, 150 seconds or less, 140 seconds or less, 130 seconds or less, 120 seconds or less, or 110 seconds or less. The time required to dissolve in water may be the time required for the crystalline kestose, added to water at a concentration of 10 wt% and stirred at 150 rpm, to be completely dissolved at 25°C.

The crystalline kestose to an embodiment of the present application is a type of oligosaccharide and has a lower sweetness than sucrose, and thus, can be used in the manufacture of mixed sweeteners, solid mixed sweeteners, chocolate, chewing gum, instant juice, instant soup, granules, tablets, and the like. In addition, the crystalline kestose may be contained and used in various compositions such as foods and beverages, favorite foods, feeds, cosmetics, and pharmaceuticals, and a method for containing the carbohydrate may be any process until the production of product is completed, and for example, a known method such as blending, mixing, dissolving, melting, immersion, penetration, spreading, applying, coating, spraying, injection, crystallization, solidification, or other known methods may be appropriately selected.

Another embodiment of the present application may provide a sweetener composition comprising the crystalline kestose. The sweetener composition may comprise kestose crystals in various contents, and may further comprise one or more selected from the group consisting of high-intensity sweeteners, monosaccharides, disaccharides, sugar alcohols, dietary fibers, and oligosaccharides.

For example, the monosaccharides and disaccharides may be one or more selected from the group consisting of allose, deoxyribose, erythrulose, galactose, idose, mannose, ribose, sorbose, tagatose, erythrose, fuculose, gentiobiose, gentiobiulose, isomaltose, isomaltulose, kojibiose, lactulose, altrose, laminaribiose, arabinose, leucrose, fucose, rhamnose, sorbose, maltulose, mannobiose, mannosucrose, melezitose, melibiose, melibiulose, nigerose, raffinose, rutinose, rutinulose, stachyose, threose, trehalose, trehalulose, turanose, xylobiose, fructose, glucose, and allulose.

The sugar alcohols may be at least one selected from the group consisting of xylitol, maltitol, erythritol, mannitol, lactitol, inositol, and sorbitol.

The dietary fibers may be water-soluble dietary fibers and the water-soluble dietary fibers may be at least one selected from the group consisting of polydextrose, digestion-resistant maltodextrin, inulin, carrageenan, guar gum, alginic acid, agar, and pectin.

The oligosaccharides may be at least one selected from the group consisting of fructooligosaccharides, isomaltooligosaccharides, maltooligosaccharides, human milk oligosaccharides, and galactooligosaccharides.

The high-intensity sweetener may be at least one selected from the group consisting of aspartame, acesulfame potassium, sodium cyclamate, sodium saccharin, sucralose, stevia sweetener (steviol glycosides, enzymatically modified stevia), dulcin, thaumatin, tomatin, neotame, rebaudiosides, Monk fruit extract, mogroside, and monellin.

### [ADVANTAGEOUS EFFECTS]

An embodiment of the present application can provide crystalline kestose having a specific particle size distribution and a large average particle size, thereby improving the hygroscopicity and dissolution rate of the kestose crystal and enhancing the storage stability.

### [BRIEF DESCRIPTION OF THE DRAWINGS]

Fig. 1 is a drawing showing an optical microscope photograph of crystalline kestose according to an embodiment of the present application.
Fig. 2 is a graph showing the moisture absorption rate of crystalline kestose according to an embodiment of the present application.
Fig. 3 is a graph showing the dissolution rate of crystalline kestose according to an embodiment of the present application.

### [MODE FOR INVENTION]

Hereinafter, the present application will be described in more detail according to the following Examples. However, these Examples are merely illustrative of the present application, and the scope of the present application is not limited to these Examples.

### Example 1: Preparation of raw solution for kestose crystallization

45 kg of water preheated to 55°C was introduced into a saccharification tank, followed by the addition of 55 kg of sucrose. The mixture was stirred for 1 to 2 hours to completely dissolve the sucrose crystals. Thereafter, the pH was adjusted to 6.5 to 7.0, and β-fructofuranosidase derived from *Aspergillus niger,* an enzyme for producing high-kestose-content syrup, was added. The mixture was allowed to react for 24 to 48 hours in accordance with the method disclosed in Korean Patent Publication No. 10-2018-0078065. At the stage where 26 wt% of sucrose remained during the enzyme reaction, the pH was adjusted to 7.6 or higher using 4N NaOH, while simultaneously heating at 80°C for 2 hours to inactivate the enzyme. Upon completion of enzyme inactivation, the mixture was decolorized/filtered, purified, and concentrated to a final concentration of 75 wt%. Subsequently, high-purity separation was performed using an SMB packed with a Na⁺-type ion exchange resin, thereby obtaining a raw solution for crystallization containing more than 85% kestose.

### < Analysis conditions for kestose content and saccharide composition >

- Analytical Equipment: HPLC Agilent, 1100 Series
- Column: Shodex Asahipak NH2P-50 4E
- Injection Volume: 10 µL
- Flow Rate: 1 mL/min
- Column Temperature: 30°C
- Mobile Phase: 70% Acetonitrile

### Example 2: Crystallization of kestose using cooling method (1)

A crystallization process was performed by slowly cooling the temperature to precipitate crystals using a raw solution for crystallization containing 91.4 wt% of kestose, 7.0 wt% of nystose, and 1.6 wt% of sucrose prepared in Example 1.

Specifically, seed crystals of 150 µm in size were added at 1 wt% based on the solid content weight to the raw solution for crystallization having an initial temperature of 60°C, pH 7.5, and a kestose solid content of 82.5 wt%. Here, the degree of supersaturation was 1.1, and the solution was cooled to 30°C at the same cooling rate of -1°C/hour. The mother liquor was removed by centrifugal dehydration, and the crystals obtained by the first crystallization were washed with cooling water and then dried to recover the kestose crystals.

The purity analysis of the produced kestose crystals was performed by HPLC analysis using the same method as in Example 1. The purity of the produced kestose crystals was 99.3 wt%, and the crystal yield was 48.4%.

### Example 3: Preparation of kestose crystal using cooling method (2)

The raw solution for crystallization containing 91.4 wt% of kestose, 7.0 wt% of nystose, and 1.6 wt% of sucrose produced in Example 1 was used to perform a crystallization process in the same manner as in Example 2, but crystallization was performed using seed crystals having a size of 300 µm.

The purity of the produced kestose crystals was analyzed by HPLC in the same manner as in Example 1, and the purity of the produced kestose crystals was 99.4 wt%, and the crystal yield was 46.6%.

### Example 4: Preparation of kestose crystal using cooling method (3)

The raw solution for crystallization containing 91.4 wt% of kestose, 7.0 wt% of nystose, and 1.6 wt% of sucrose produced in Example 1 was used to perform a crystallization process in the same manner as in Example 2, except that 0.5 wt% of seed crystals of 300 µm in size were added based on the solid content, and the cooling rate was -0.5°C/hour to 30°C at the same rate.

The purity of the produced kestose crystals was analyzed by HPLC in the same manner as in Example 1, and the purity of the produced kestose crystals was 99.7 wt%, and the crystal yield was 51.6%.

### Example 5: Preparation of kestose crystal using evaporative concentration Method (1)

By using the raw solution for crystallization containing 91.4 wt% of kestose, 7.0 wt% of nystose, and 1.6 wt% of sucrose produced in Example 1, a kestose-containing solution having a solid content of 68 wt% and a pH of 7.5 was introduced into the reactor to occupy 25% of its volume at a temperature of 60°C in a vacuum state, and concentrated under vacuum to a solid content of 82.5 wt%.

The seed crystals were introduced at the raw solution concentration with a supersaturation degree of 1.1, and slowly stirred to generate crystal nuclei. While maintaining the vacuum state, the raw solution was additionally introduced in the same volume as that of the initial solution, and the crystals were grown while the concentration was performed to maintain the solid content of 82.5 wt% and the supersaturation degree constant. At this time, without lowering the temperature, the solution was added under constant temperature conditions and concentrated to a solid content of 82.5 wt% so that the supersaturation degree of 1.1 could be maintained. Since fine crystals might also precipitate as the crystals grow, a small amount of water was added to dissolve the fine crystals prior to the addition of the raw solution, and then the raw solution was added to prevent the crystal particle growth from being inhibited by the fine crystals. The method of adding the raw solution and adding the water was repeated a total of 4 times as described above.

After discharging the produced kestose crystals from the reactor, the mother liquor was removed by centrifugal dehydration, and the crystals obtained by the first crystallization were washed with cooling water and then dried to recover them. The purity analysis of the produced kestose crystals was performed by HPLC analysis in the same manner as in Example 1, and the purity of the produced kestose crystals was 99.7 wt% and the crystal yield was 56.8%.

In the case of Examples 4 and 5, since the pH of the raw solution was 7.5, the content of kestose did not decrease even though the crystallization process was performed at high temperature for a long time, and the content of nystose, which could act as an inhibitor of kestose crystal growth, was less than 7 wt%, so it did not significantly affect the crystallization process.

### Example 6: Preparation of kestose crystal using evaporative concentration Method (2)

The raw solution for crystallization containing 91.4 wt% of kestose, 7.0 wt% of nystose, and 1.6 wt% of sucrose produced in Example 1 was prepared and the pH was adjusted to 4.3 using 1N HCI. In the same manner as Example 5, a kestose-containing solution having a solid content of 68 wt% was introduced at a temperature of 65°C in a vacuum state to make 25% of the reactor volume, and vacuum concentration was performed in the reactor to make the solid content 82.5 wt%.

The seed crystals were added at a supersaturation degree of 1.1 and stirred slowly to generate crystal nuclei. While maintaining the vacuum state, the raw solution was additionally added in the same volume as that of the initial solution, and crystallization was carried out under concentration conditions that maintained a solid content of 82.5 wt% and a constant level of supersaturation to promote crystal growth. However, the crystals in the raw solution for crystallization did not grow well, resulting in the inclusion of many fine crystals were included. In order to grow the fine crystals into larger crystals, a small amount of water was added to re-dissolve the fine crystals before adding the raw solution, and then the raw solution was added, but the crystals did not grow well.

When the raw solution during the crystallization was sampled and the saccharide composition was analyzed, the pH was below 5, and the kestose and nystose components were decomposed by the high-temperature reaction for a long time, so the kestose content was somewhat reduced to 88.8%. It was determined that a composition containing 10% or more of the nystose component would be a content that would hinder the growth of the kestose crystals. Therefore, the kestose crystals were not recovered in Example 6.

Therefore, it was determined that a pH of 5 or higher and a nystose content of 10% or less of the total saccharide composition are required for kestose crystallization.

### Comparative Example 1: Separation of kestose crystal particles

The crystals passing through 100 mesh obtained by sieving the kestose crystals prepared in Example 2 and the crystals obtained in Example 4 were mixed in a ratio of 7.7:2.3 to produce a kestose crystal sample having an average particle size of about 200 µm and a high content of fine particles.

### Test Example 1: Observation of kestose crystal morphology

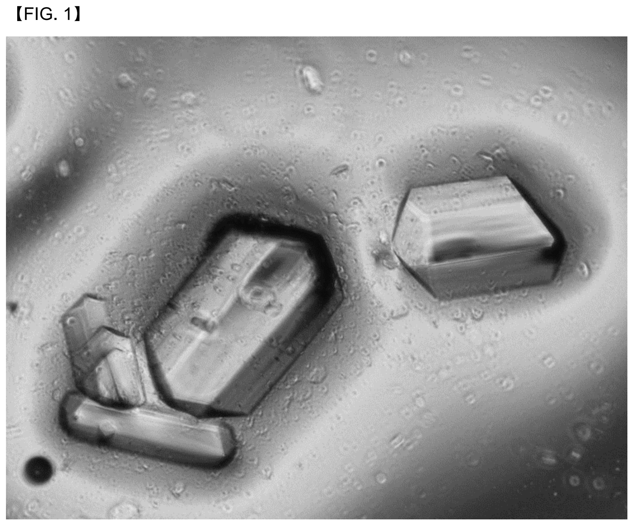

The morphology of the Kestose crystals manufactured in Example 4 was observed using an optical microscope. Fig. 1 is an optical microscope photograph of the kestose crystal particles manufactured in Example 3 measured at a magnification of X100. As shown in Fig. 1, it was found that the kestose crystals according to the present application had a rectangular hexahedral shape, indicating high uniformity and hardness of the crystals.

### Test Example 2: Analysis of particle size distribution of kestose crystals

In order to confirm the particle size distribution of kestose crystals manufactured in Examples 2 to 5 and Comparative Example 1, a particle size analysis device using a laser diffraction method was used. The particle size distribution was analyzed by repeating the sample several times. The results are shown in Table 1.

### <Analysis conditions>

Analyzer: Mastersizer2000 (MALVERN Instrument)
Accessory Equipment Name: Hydro 2000MU (A)
Dispersant: Isopropyl alcohol

**[Table 1]**

| ITEM | Example2 | Example 3 | Example 4 | Example 5 | Comparative Example 1 |
|---|---|---|---|---|---|
| D[4, 3] - Volume weighted mean (µm) | 200.6 | 282.2 | 357.1 | 345.6 | 201.7 |
| 10 µm or less(%) | 1.23 | 1.02 | 0.34 | 0.36 | 5.87 |
| 20 µm or less (%) | 2.21 | 2.18 | 0.94 | 0.92 | 7.95 |
| 50 µm or less (%) | 6.41 | 4.58 | 2.65 | 2.69 | 21.45 |
| 60 µm or less (%) | 7.66 | 5.17 | 3.11 | 3.18 | 26.34 |
| 70 µm or less (%) | 10.40 | 7.35 | 4.60 | 4.67 | 36.95 |
| 80 µm or less(%) | 11.98 | 9.28 | 5.82 | 5.88 | 42.03 |
| 100 µm or less(%) | 13.91 | 12.01 | 7.49 | 7.55 | 46.70 |
| 140 µm or less(%) | 25.59 | 25.43 | 16.07 | 16.45 | 59.82 |
| 180 µm or less(%) | 32.84 | 31.25 | 20.20 | 20.91 | 64.76 |
| 200 µm or less(%) | 42.13 | 37.32 | 24.90 | 26.05 | 69.93 |
| 240 µm or less(%) | 65.42 | 49.24 | 35.77 | 38.02 | 78.65 |
| more than 240 µm (%) | 34.58 | 50.76 | 64.23 | 61.98 | 21.35 |

As shown in Table 1, when preparing kestose crystals by the cooling crystallization method, the larger the size of seed crystals, the larger the particle size distribution of the final kestose crystal particles. This means that the size of seed crystals is an important factor for the growth of kestose crystals. In addition, the growth pattern of kestose crystals could be controlled by controlling the amount of seed crystals and the cooling rate. Therefore, it was confirmed that the amount of seed crystals, the size of the seed crystals added, and the cooling rate are factors affecting kestose crystallization.

### Test Example 4: Differential Scanning Calorimetry (DSC) Analysis

DSC analysis of the Kestose crystals prepared in Example 4 was performed, and fructooligosaccharide powder containing 85% Kestose manufactured by CVD drying was used as a control group. The specific conditions of DSC analysis were as follows:
- Equipment name: DSC [differential scanning calorimetry]
- Manufacturer: Perkin Elmer
- Method: 30 to 250°C, 10°C/min heating, N2 gas purge (Standard method: Refer to ASTM D 3418)

The DSC analysis results of the kestose crystals are shown in Table 2.

**[Table 2]**

| Item | Tm(°C) | ΔH(J/g) |
|---|---|---|
| Example 4 | 206.35 | 120.1 |
| Fructoolgosaccharide powder (containing 85% of kestose) | 85.06 | 55.70 |

As a result of DSC analysis, the Kestose crystal according to an example of the present application was measured to have a relatively high Tm value and high heat capacity compared to an amorphous powder manufactured directly by a spray dryer or a CVD method without crystallization. In the DSC analysis of a crystal, the higher the heat capacity, the more difficult it is to melt, and it can be predicted that the higher the heat capacity and the narrower the width of the endothermic peak and the more uniformly and firmly the crystal is formed. Therefore, it was confirmed that by preparing crystalline particles through crystallization, Kestose could be formed more uniformly and firmly than an amorphous powder product and could be used stably.

### Test Example 5: Measurement of hygroscopicity of Kestose crystals

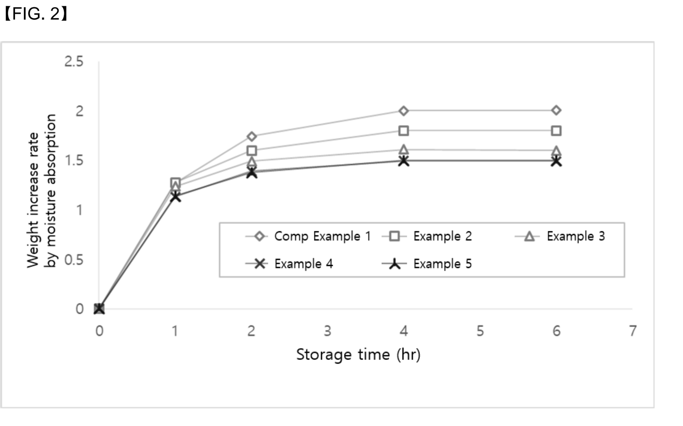

A hygroscopicity comparison test of the kestose crystals obtained in Examples 2 to 5 and Comparative Example 1 was conducted. Specifically, in order to quickly compare the effects on hygroscopicity, a constant temperature and humidity chamber set to 25°C and 75% relative humidity was used. The weight of each sample was measured exactly 10g, and the sample was stored over time under constant temperature and humidity conditions. The increased weight was measured from the total weight of the plate including the initial sample, and the moisture absorption was determined by the increased weight. The increased weight was calculated as a percentage based on the initial combined weight of the plate and the sample. A hygroscopicity comparison graph of each sample is shown in Fig. 2, and the increase rate (%) of the hygroscopic weight of each sample is shown in Table 3.

As shown in Table 3, the kestose crystals of Examples 2 and 3 showed rate of weight increase by moisture absorption of 1.8% and 1.6%, respectively, during a 6-hour storage period, and the kestose crystals of Examples 4 and 5 showed weight increase rate by moisture absorption of about 1.5%. On the other hand, the kestose crystal of Comparative Example 1 showed a large weight increase rate of more than 2%. As a result, it was confirmed that as the crystal particles of kestose grew, kestose with greatly improved hygroscopicity could be provided.

**[Table 3]**

| Storage time(hr) | Comparative Example 1 Weight increase rate (%) | Example 2 Weight increase rate (%) | Example 3 Weight increase rate (%) | Example 4 Weight increase rate (%) | Example 5 Weight increase rate (%) |
|---|---|---|---|---|---|
| 0 | 0 | 0 | 0 | 0 | 0 |
| 1 | 1.27 | 1.28 | 1.23 | 1.13 | 1.14 |
| 2 | 1.74 | 1.60 | 1.49 | 1.39 | 1.38 |
| 4 | 2.00 | 1.80 | 1.61 | 1.49 | 1.50 |
| 6 | 2.01 | 1.80 | 1.60 | 1.49 | 1.50 |

### Test Example 6: Measurement of dissolution rate of kestose crystals

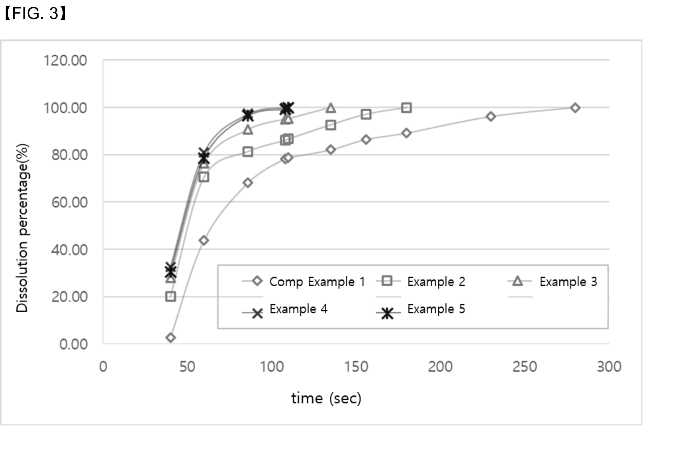

A dissolution rate comparison test of kestose crystals manufactured in Examples 2 to 5 and Comparative Example 1 was conducted. Specifically, 20 g of the sample was added to 180 g of water, and the time until complete dissolution was measured. The sample was stirred at a constant speed of 150 rpm at 25°C, and the solid content of the supernatant was measured to calculate the solubility until the final dissolution concentration was reached as a percentage, and the time of complete dissolution was confirmed. A graph comparing the dissolution rates of each sample is shown in Fig. 3, and the solubility percentage (%) of each sample is shown in Table 4.

The times until the kestose crystals of Examples 2 and 3 were completely dissolved were 180 seconds and 135 seconds, respectively, and the kestose crystals of Examples 4 and 5 took 108 seconds and 110 seconds, respectively, to be completely dissolved. On the other hand, the kestose crystal of Comparative Example 1 took the longest time to completely dissolve, taking 280 seconds.

Generally, when the crystal size is large, the dissolution speed is slow. However, the kestose crystal according to an embodiment of the present application showed a fast dissolution speed because the time to completely dissolve was short despite having a large average particle size. In addition, the kestose crystal according to an embodiment of the present application showed a faster dissolution speed as the size increased. This is a characteristic different from the characteristics of general crystals. The kestose crystal is a trisaccharide crystal, and has the characteristic that the viscosity of the crystal solution is high and agglomeration easily occurs during the crystallization process. However, the kestose crystal according to an embodiment of the present application was determined to have a high dissolution speed because the average particle size is large and the distribution ratio of fine crystals that are easy to aggregate is small. Therefore, an embodiment of the present application can provide a kestose having reduced agglomeration phenomenon between crystals during dissolution and improved dissolution speed.

**[Table 4]**

| Time (sec) | Comparative Example 1 Dissolution percentage(%) | Example 2 Dissolution percentage (%) | Example 3 Dissolution percentage (%) | Example 4 Dissolution percentage (%) | Example 5 Dissolution percentage (%) |
|---|---|---|---|---|---|
| 40 | 2.70 | 20.00 | 28.10 | 32.43 | 30.48 |
| 60 | 43.96 | 70.65 | 76.30 | 81.08 | 78.65 |
| 86 | 68.16 | 81.40 | 90.60 | 97.30 | 96.45 |
| 108 | 78.40 | 86.10 | 95.10 | 100 | 99.21 |
| 110 | 78.80 | 86.70 | 95.30 | 100 | 100 |
| 135 | 82.10 | 92.70 | 100 | 100 | 100 |
| 156 | 86.50 | 97.20 | 100 | 100 | 100 |
| 180 | 89.20 | 100 | 100 | 100 | 100 |
| 230 | 96.20 | 100 | 100 | 100 | 100 |
| 280 | 100 | 100 | 100 | 100 | 100 |

## Claims

1. A crystalline kestose having a particle size distribution in which an average particle size represented by D(4,3) is 200 µm or more, and particles having a size of 70 µm or less account for 35% or less.

2. The crystalline kestose according to claim 1, having a particle size distribution in which the average particle size represented by D(4,3) is 200 µm or more,
particles having a size of 50 µm or less account for 20% or less, and
particles having a size of 70 µm or less account for 35% or less.

3. The crystalline kestose according to claim 1, further having at least a particle size distribution selected from the group consisting of the following (1) to (10):
(1) particles having a size of 10 µm or less account for 5.5% or less,
(2) particles having a size of 20 µm or less account for 7.5% or less,
(3) particles having a size of 50 µm or less account for 20% or less,
(4) particles having a size of 60 µm or less account for 25% or less,
(5) particles having a size of 80 µm or less account for 42% or less,
(6) particles having a size of 100 µm or less account for 45% or less,
(7) particles having a size of 140 µm or less account for 55% or less,
(8) particles having a size of 180 µm or less account for 60% or less,
(9) particles having a size of 200 µm or less account for 65% or less, and
(10) particles having a size of 240 µm or less account for 78% or less.

4. The crystalline kestose of claim 1, wherein the crystalline kestose has a melting temperature (Tm) of 206±5°C.

5. The crystalline kestose of claim 1, wherein the crystalline kestose has a melting enthalpy (△H) of 120±5 J/g.

6. The crystalline kestose of claim 1, wherein the crystalline kestose has a purity of 70 wt% or more.

7. The crystalline kestose of claim 1, wherein the crystalline kestose contains less than 1.4 wt% of nystose.

8. The crystalline kestose of claim 1, wherein the crystalline kestose has a hygroscopicity of 2% or less when stored at a temperature of 25°C and a relative humidity of 75% for 6 hours.

9. The crystalline kestose of claim 1, wherein the crystalline kestose takes 250 seconds or less to dissolve in water to be at a concentration of 10% (w/w).

10. A fructooligosaccharide composition comprising the crystalline kestose according to any one of claims 1 to 9.

11. A method for producing the crystalline kestose according to any one of claims 1 to 9, comprising
a step of generating crystal nuclei at a temperature where the supersaturation degree of a kestose solution having a nystose content of less than 10 wt% based on a solid content of 100 wt% and a pH of 5 or higher, is greater than 1 and less than 1.4; and
a step of growing crystals.

12. The method according to claim 11, wherein the kestose content of the kestose solution is 80 wt% or more based on a solid content of 100 wt%.

13. The method according to claim 11, wherein the step of growing crystals is performed by growing crystals by cooling the kestose solution.

14. The method according to claim 13, wherein the cooling is not accompanied by concentration under reduced pressure.

15. The method according to claim 13, wherein the cooling is performed at a rate of 5°C or less per hour.

16. The method according to claim 13, wherein the kestose solution is cooled to a temperature of 30 to 75°C.

17. The method according to claim 11, wherein the step of growing crystals grows the crystals by concentrating the kestose-containing raw solution for crystallization.

18. The method according to claim 17, wherein the kestose-containing raw solution for crystallization is concentrated to a solid content of 60 to 90 wt%.

19. The method according to claim 17, wherein the concentrating is not accompanied by a decrease in temperature.

20. The method according to claim 11, wherein the step of growing crystals further comprises a step of dissolving fine crystals produced in the step of growing crystals one or more time.

21. The method according to claim 11, further comprising a step of adding a seed crystal.

22. The method according to claim 11, wherein the crystallization yield of the method is 40% or more.
